# EUROPEAN PATENT APPLICATION

(11) **EP 3 954 755 A1**
(43) Date of publication of application: **16.02.2022**
(21) Application number: 20770513.8
(22) Date of filing: 02.03.2020
(51) Int. Cl.: C12N 1/20, C12Q 1/04

(54) **SELECTION MEDIUM FOR BACTERIA BELONGING TO GENUS PSEUDOMONAS AND USE THEREOF**

(30) Priority: 08.03.2019 JP 2019042297
(71) Applicant: Kabushiki Kaisha Yakult Honsha, Tokyo, 105-8660 (JP)
(72) Inventor: NIIKURA Mai, Tokyo 105-8660 (JP); ASAHARA Takashi, Tokyo 105-8660 (JP); TAKAHASHI Akira, Tokyo 105-8660 (JP)
(74) Representative: Schiener, Jens
(86) International application number: PCT/JP2020/008581
(87) International publication number: WO 2020/184236

(57) **Abstract**

A bacterium belonging to the genus *Pseudomonas* such as *Pseudomonas aeruginosa* is specifically and quantitatively detected using a selection medium for a bacterium belonging to the genus *Pseudomonas* characterized by containing, in a basal medium, at least one third-generation cephalosporin and at least one antibiotic selected from the group consisting of a first-generation cephalosporin and a glycopeptide-based antibiotic and by a method for detecting a bacterium belonging to the genus *Pseudomonas* and a method for selecting a bacterium belonging to the genus *Pseudomonas* through use of said selection medium.

## Description

### Technical Field

The present invention relates to a selection medium for a bacterium belonging to the genus *Pseudomonas* and a method for detecting a bacterium belonging to the genus *Pseudomonas* and a method for selecting a bacterium belonging to the genus *Pseudomonas* using the selection medium.

### Background Art

*Pseudomonas aeruginosa,* which is a bacterium belonging to the genus *Pseudomonas,* is a bacterium which involves a very low risk of causing infectious diseases in healthy individuals but is a bacterium that causes postoperative infections, ventilator-associated pneumonia or severe bacteremias in patients with decreased resistance due to surgery, cancer therapy, graft surgery or the like.

To detect bacteria belonging to the genus *Pseudomonas* in general medical facilities, a culture method is used (NPL 1). However, the selection media used for the culture method, such as CHROM plate medium and NAC plate medium, sometimes also detect a bacterium other than the bacteria belonging to the genus *Pseudomonas,* and the selection factors contained in the media act antibacterially also on the bacteria belonging to the genus *Pseudomonas* and estimate the viable cell count low. Therefore, although qualitative inspection is possible, it is difficult to assess the degree of infection or pollution with the bacterial count.

Accordingly, for appropriate selection of an antibacterial agent and early therapy through the culture method, a novel selection medium which can specifically and accurately quantify a bacterium belonging to the genus *Pseudomonas* has been required.

Moreover, regarding *Pseudomonas aeruginosa* in particular, of bacteria belonging to the genus *Pseudomonas,* it is reported that *1) Pseudomonas aeruginosa* is isolated highly frequently not only from the blood and the sputa of inpatients but also from their feces and that 2) there are many subclinical infection patients (patients who are carriers and who do not develop the infection) and carrier transmission occurs easily from the intestinal tract as the source of infection. Thus, quantitative detection of *Pseudomonas aeruginosa* in feces can greatly contribute also to the control of the spread of infection.

### Citation List

### Non Patent Literature

NPL 1: Sigurdardottir K, Digranes A, Harthug S, Nesthus I, Tangen JM, Dybdahl B, Meyer P, Hopen G, Lokeland T, Grottum K, Vie W, Langeland N. (2005) A multi-centre prospective study of febrile neutropenia in Norway: microbiological findings and antimicrobial susceptibility. Scand J Infect Dis 37: 455-64.

### Summary of Invention

### Technical Problem

In view of the above points, a problem of the present invention is to provide a selection medium which specifically and quantitatively detects a bacterium belonging to the genus *Pseudomonas* such as *Pseudomonas aeruginosa.*

### Solution to Problem

As a result of intensive studies to solve the above problem, the present inventors have found that a bacterium belonging to the genus *Pseudomonas* can be selectively cultured by adding specific antibiotics in combination to a basal medium and thus completed the present invention.

That is, the present invention relates to a selection medium for a bacterium belonging to the genus *Pseudomonas* characterized by containing, in a basal medium, at least one third-generation cephalosporin and at least one antibiotic selected from the group consisting of a first-generation cephalosporin and a glycopeptide-based antibiotic.

The present invention also relates to a method for detecting a bacterium belonging to the genus *Pseudomonas* characterized by seeding and culturing a sample in the selection medium for a bacterium belonging to the genus *Pseudomonas* and detecting the bacterium belonging to the genus *Pseudomonas.*

The present invention also relates to a method for selecting a bacterium belonging to the genus *Pseudomonas* characterized by seeding and culturing a sample in the selection medium for a bacterium belonging to the genus *Pseudomonas* and selectively culturing the bacterium belonging to the genus *Pseudomonas.*

### Advantageous Effects of Invention

Because a bacterium belonging to the genus *Pseudomonas* such as *Pseudomonas aeruginosa* can be selectively cultured in the selection medium for a bacterium belonging to the genus *Pseudomonas* of the present invention, the bacterium belonging to the genus *Pseudomonas* can be specifically and quantitatively detected from a sample.

Accordingly, the present invention can be used for appropriate selection of an antibacterial agent and early therapy in medical facilities.

### Description of Embodiments

The selection medium for a bacterium belonging to the genus *Pseudomonas* of the present invention (referred to as "the medium of the present invention" below) contains, in a basal medium, at least one third-generation cephalosporin and at least one antibiotic selected from the group consisting of a first-generation cephalosporin and a glycopeptide-based antibiotic.

The basal medium used for the medium of the present invention is not particularly limited as long as the medium does not inhibit the growth of the bacterium belonging to the genus *Pseudomonas,* and examples thereof include NAC medium, TSA medium and the like. Although one kind or a combination of two or more kinds of basal medium can be used, a medium mixture of NAC medium and a medium other than NAC medium which does not inhibit the growth of the bacterium belonging to the genus *Pseudomonas* is preferable in view of the selectivity, and a medium mixture of NAC medium and TSA medium is more preferable in view of the selectivity. The mixing ratio of NAC medium and TSA medium is not particularly limited, but the mass ratio is preferably 1:1 to 40, particularly preferably 1:1 to 20, more preferably 1:10 to 20. The compositions of NAC medium and TSA medium are as follows.

**TSA (Trypticase Soy Agar) Medium**

| | |
|---|---|
| peptone from casein pancreatically digested | 15.0 g/l |
| peptone from soymeal papain-digested | 5.0 g/l |
| sodium chloride | 5.0 g/l |
| agar | 15.0 g/l |

**NAC (Nalidic-Acid, Cetrimide) Medium**

| | |
|---|---|
| peptone | 20.0 g/l |
| potassium hydrogen phosphate | 0.3 g/l |
| magnesium sulfate | 0.2 g/l |
| cetrimide | 0.2 g/l |
| nalidixic acid | 0.015 g/l |
| agar | 15.0 g/l |

The third-generation cephalosporin used for the medium of the present invention is not particularly limited, but examples thereof include ceftizoxime, cefotaxime, ceftriaxone, ceftazidime, cefoperazone, cefsulodin, ceftibuten, cefixime, cefetamet, cefditoren pivoxil and the like. Of the third-generation cephalosporins, ceftizoxime and/or cefotaxime is preferable, and ceftizoxime or cefotaxime is more preferable. Ceftizoxime is particularly preferable.

The first-generation cephalosporin used for the medium of the present invention is not particularly limited, but examples thereof include cefalotin, cephazolin/cefazolin, cephapirin, cephalexin, cephradine, cephadroxil and the like. Of the first-generation cephalosporins, cefalotin is preferable.

The glycopeptide-based antibiotic used for the medium of the present invention is not particularly limited, but examples thereof include vancomycin, teicoplanin and the like. Of the glycopeptide-based antibiotics, vancomycin is preferable.

In the present specification, the names of the antibiotics cited as the examples of the third-generation cephalosporin, the first-generation cephalosporin and the glycopeptide-based antibiotic are all general names, and hydrochlorides thereof, sodium salts thereof and the like are also included.

The third-generation cephalosporin, first-generation cephalosporin and glycopeptide-based antibiotic contents of the medium of the present invention may be appropriately determined considering the minimum inhibitory concentrations of the bacterium belonging to the genus *Pseudomonas* and another bacterium and is not particularly limited. For example, the third-generation cephalosporin content is 0.1 µg/ml or more and less than 64 µg/ml, preferably 0.5 µg/ml or more and 32 µg/ml or less. For example, the first-generation cephalosporin content is 16 µg/ml or more, preferably 32 µg/ml or more and 256 µg/ml or less. For example, the glycopeptide-based antibiotic content is higher than 128 µg/ml, preferably higher than 128 µg/ml and 256 µg/ml or less.

More specifically, combinations of at least one third-generation cephalosporin and at least one antibiotic selected from the group consisting of a first-generation cephalosporin and a glycopeptide-based antibiotic are those described below.

### <Combination 1>

ceftizoxime 0.1 µg/ml or more and less than 64 µg/ml
cefalotin 32 µg/ml or more and 256 µg/ml or less

### <Combination 2>

cefotaxime 0.1 µg/ml or more and less than 16 µg/ml
cefalotin 32 µg/ml or more and 256 µg/ml or less

### <Combination 3>

ceftizoxime 0.1 µg/ml or more and less than 64 µg/ml
vancomycin higher than 128 µg/ml and 256 µg/ml or less

### <Combination 4>

cefotaxime 0.1 µg/ml or more and less than 16 µg/ml
vancomycin higher than 128 µg/ml and 256 µg/ml or less

A preferable embodiment of the medium of the present invention is a medium which is obtained by mixing NAC medium and TSA medium at a mass ratio of 1:1 to 20 and which contains ceftizoxime at 0.1 µg/ml or more and less than 64 µg/ml, cefalotin at higher than 16 µg/ml and vancomycin at a concentration higher than 128 µg/ml.

A more preferable embodiment of the medium of the present invention is a medium which is obtained by mixing NAC medium and TSAmedium at a mass ratio of 1:1 to 20 and which contains ceftizoxime at 0.1 µg/ml or more and less than 64 µg/ml and cefalotin at 16 µg/ml or more.

The medium of the present invention may contain, for example, yeast extract, minerals, vitamins and the like as long as the effects of the present invention are not impaired.

The method for producing the medium of the present invention is not particularly limited, and the medium can be produced as follows, for example.

First, a basal medium which has been heated and dissolved is sterilized with an autoclave or the like. Next, after the sterilization, at least one third-generation cephalosporin and at least one antibiotic selected from the group consisting of a first-generation cephalosporin and a glycopeptide-based antibiotic are added to the basal medium at desired concentrations, and the mixture is stirred. In the end, the medium is dispensed in containers and dried until the medium solidifies.

The medium of the present invention obtained in this manner can specifically and quantitatively detect a bacterium belonging to the genus *Pseudomonas* such as *Pseudomonas aeruginosa, P. pseudoalcaligenes, P. stutzeri, P. luteola, P. oryzihabitans, P. putida, P. tolaasii, P. alcaligenes, P. fluorescens, P. paucimobilis* and *P. acidovorans,* preferably *Pseudomonas aeruginosa.* Here, the term specifically means that bacteria contained in the seeded sample except for *Pseudomonas aeruginosa* are not detected and that only *Pseudomonas aeruginosa* is detected. Moreover, the term quantitatively here means that the accurate bacterial count of *Pseudomonas aeruginosa* contained in the seeded sample can be measured.

The medium of the present invention has the above characteristics and can be thus used for a method for detecting a bacterium belonging to the genus *Pseudomonas* and a method for selecting a bacterium belonging to the genus *Pseudomonas.*

The method for detecting a bacterium belonging to the genus *Pseudomonas* of the present invention (referred to as "the detection method of the present invention" below) is conducted by seeding and culturing a sample in the medium of the present invention and detecting the bacterium belonging to the genus *Pseudomonas.*

The sample used for the detection method of the present invention is not particularly limited as long as the sample is supposed to contain the bacterium belonging to the genus *Pseudomonas,* but examples thereof include feces, blood, sputa and the like. Of these samples, feces contain a large number of bacteria, and it is very difficult to specifically detect only a bacterium belonging to the genus *Pseudomonas* from the bacteria. However, using the medium of the present invention, the bacterium belonging to the genus *Pseudomonas* only can be specifically detected. Such a sample is obtained by diluting feces 10 times with TSB medium, physiological saline or the like, and 100 µl thereof is seeded and cultured in the medium of the present invention. The culture conditions are not particularly limited, but an example thereof is aerobic culture at 37°C for 24 hours. The bacterium belonging to the genus *Pseudomonas* is specifically cultured after such culture, and the bacterium belonging to the genus *Pseudomonas* can be thus detected.

The method for selecting a bacterium belonging to the genus *Pseudomonas* of the present invention (referred to as "the selection method of the present invention" below) is conducted by seeding and culturing a sample in the medium of the present invention and selectively culturing the bacterium belonging to the genus *Pseudomonas.*

The sample used for the selection method of the present invention is not particularly limited as long as the sample is supposed to contain the bacterium belonging to the genus *Pseudomonas,* but examples thereof include feces, blood, sputa and the like. Of these samples, feces are preferable. Such a sample is obtained by diluting feces 10 times with TSB medium, physiological saline or the like, and 100 µl thereof is seeded and cultured in the medium of the present invention. The culture conditions are not particularly limited, but an example thereof is aerobic culture at 37°C for 24 hours. The bacterium belonging to the genus *Pseudomonas* is specifically cultured after such culture, and the bacterium belonging to the genus *Pseudomonas* can be thus selected.

### Examples

The present invention is explained in detail below referring to Examples, but the present invention is not limited to the Examples.

### <Used Bacterial Line and Culture Conditions>

In the following Examples, *Pseudomonas aeruginosa* ATCC10145^{T} (standard line: acquired from ATCC, 10801 University Boulevard Manassas, VA 20110 USA: referred to as "*P*. *aeruginosa"* below) was used as *Pseudomonas aeruginosa. P. aeruginosa* which was frozen and stored at -80°C (beads stock, MICROBANK^{™}, PRO-LAB Diagnostics) was seeded in 10 ml of Trypticase soy broth (TSB medium: Difco) and shaken-cultured (140 rpm) at 37°C for 18 hours under aerobic conditions. The culture solution was sucked up and discharged 10 times with a syringe with a needle having an internal size of 0.27 mm (homogenization) and then used.

### Example 1

Determination of Composition of Novel Selection Medium:

### (1) Examination of Addition Ratio of NAC Medium and TSA Medium

NAC (Nalidic-Acid, Cetrimide) medium, which is used for clinical tests as a medium for detecting *P. aeruginosa,* was used as the basal medium of a novel selection medium, and the concentration of NAC medium at which the growth of *P. aeruginosa* is not inhibited was examined. That is, media were produced by appropriately diluting NAC medium (Nissui Pharmaceutical Co., Ltd.) with a non-selection medium, Trypticase Soy Agar medium (TSA medium: Difco) (NAC medium:TSA medium = 1:1, 1:2, 1:10, 1:20 and 1:40), and a bacterial solution of *P. aeruginosa* which was prepared at 10⁴ CFU/ml using 1% Tween20-added physiological saline (after homogenization) was seeded to the plate media (duplicate). The plate media were aerobically cultured at 37°C for 24 hours. The numbers of colonies appeared on the media were counted, and the inhibition rates of the growth of *P. aeruginosa* were determined. The results are shown in Table 1.

**[Table 1]**

| Medium | NAC medium:TSA medium | | | | | |
|---|---|---|---|---|---|---|
| | 1:0 | 1:1 | 1:2 | 1:10 | 1:20 | 1:40 |
| Inhibition Rate (%) | 97.0 | 18.8 | 17.6 | 11.8 | 0 | 0 |

NAC medium inhibited the growth of *P. aeruginosa* by 97%. When TSA medium was added to NAC medium at a ratio of 1:20, the growth of *P. aeruginosa* was not inhibited at all any more. From the results, it was decided that a medium obtained by mixing NAC medium and TSA medium at a ratio of 1:20 is used as the basal medium of a novel selection medium.

### (2) Identification of Bacteria in Feces Growing in TSA Medium-Diluted NAC Medium

After mixing feces obtained from three healthy volunteers into one, a 10× suspension of the feces was produced by adding nine times the amount of TSB medium and vigorously stirring the mixture. On a plate medium produced by mixing NAC medium and TSA medium at a ratio of 1:20, 100 µl taken from the suspension was seeded and aerobically cultured at 37°C for 24 hours. The colonies appeared on the medium were subjected to colony PCR, and the sequences of the obtained amplified products were analyzed and subjected to homology search on database. The bacterial kinds having colony-forming capability on the medium were identified.

It was observed that *Escherichia coli* (referred to as "*E*. *coli"* below), *Enterococcus faecium* (referred to as "*E*. *faecium"* below) and *Comamonas kerstersii* (referred to as "*C*. *kerstersii"* below) in the feces have colony-forming capability on the plate medium obtained by mixing NAC medium and TSA medium at a ratio of 1:20.

### (3) Examination of Antibacterial Agents Which Inhibit Growth of Bacteria in Feces Other Than P. aeruginosa

### (3-1) Preparation of Bacterial Solutions

Colonies of *E. coli, E. faecium* and *C*. *kerstersii,* which formed colonies on the plate medium obtained by mixing NAC medium and TSA medium at a ratio of 1:20, and colonies of *P. aeruginosa* were taken, added to 500 µl of the basal medium, stirred and subjected to the following experiment.

### (3-2) Preparation of Antibacterial Agents

As antibacterial agents to which the sensitivity of *P*. *aeruginosa* is low and to which the sensitivities of *E. coli, E. faecium* and *C*. *kerstersii* are high, cefotaxime sodium salt (Sigma (referred to as "Cefotaxime" below)), ceftizoxime sodium (Fujisawa Healthcare (referred to as "Ceftizoxime" below)), vancomycin hydrochloride (Sigma (referred to as "Vancomycin" below)), minocyclin hydrochloride (Sigma (referred to as "Minocyclin" below)) and cefalotin sodium salt (Sigma (referred to as "Cefalotin" below)) were selected. The antibacterial agents were dissolved in sterilized distilled water at 51.2 mg/ml and then sterilized by filtration through a membrane filter of 0.45 µm (DISMIC-25cs: Toyo Roshi) .

### (3-3) Measurement of Minimum Inhibitory Concentrations (MICs)

The aqueous antibacterial agent solutions after the filtration sterilization were added to the basal medium (Trypticase Soy Broth: Difco) to a concentration of 512 µg/ml and further diluted with the basal medium to produce a two-fold serial dilution of 0.5 to 256 µg/ml. The antibacterial agent-added liquid media having concentrations of 0.5 to 256 µg/ml were dispensed each in a volume of 100 µl to the wells of a 96-well plate.

After adding 5 µl of the bacterial solutions to the wells and thoroughly stirring, the bacteria were cultured at 37°C for 24 hours . After culturing, the OD values (660 nm) were measured, and the lowest concentrations of the antibacterial agents at which the growth was inhibited as compared to that of the negative control (MICs: minimum inhibitory concentrations) were determined. As the negative control, the liquid medium to which the antibacterial agents were not added was used. The results are shown in Table 2.

**[Table 2]**

| | MIC (µg/ml) | | | |
|---|---|---|---|---|
| | *P. aeruginosa* | *E. coli* | *E. faecium* | *C. kerstersii* |
| Cefotaxime | 16 | <0.5 | NT | NT |
| Ceftizoxime | 64 | <0.5 | NT | NT |
| Vancomycin | 256< | NT | 8 | 128 |
| Minocyclin | <0.5 | NT | <0.5 | <0.5 |
| Cefalotin | 256< | NT | 8 | 16 |

| | | | | |
|---|---|---|---|---|
| NT: Measurement was not conducted. | | | | |

Cefotaxime and Ceftizoxime strongly inhibited the growth of *E. coli* (MIC: <0.5 µg/m), but the MIC value of Ceftizoxime with respect to *P. aeruginosa* was higher. Therefore, it was decided to use Ceftizoxime, and the addition concentration thereof was determined to be 0.5 µg/ml, at which the growth of *E. coli* is inhibited and which is lower than the MIC value of *P. aeruginosa.* It was found that, although Vancomycin was not suitable for use at a low concentration because the sensitivity of C. *kerstersii* was low, Vancomycin can be used when the concentration thereof is higher than 128 µg/ml. Minocyclin was not suitable for use because *P. aeruginosa* was sensitive. On the other hand, to Cefalotin, only the sensitivity of *P. aeruginosa* was low (MIC: >256 µg/ml) as compared to those of the other tested bacteria. The addition concentration of Cefalotin was determined to be 64 µg/ml, at which the growth of *P. aeruginosa* is not inhibited and at which the growth of *E. faecium* and *C*. *kerstersii* can be inhibited.

### Example 2

Assessment of Properties of Newly Produced Selection Medium:

### (1) Production of Tested Media

Three kinds of plate media, namely NAC medium, a selection medium of the present invention (NCC medium) which was obtained by mixing NAC medium and TSA medium at a ratio of 1:20 and adding Ceftizoxime (final concentration: 0.5 µg/ml) and Cefalotin (final concentration: 64 µg/ml), which inhibit the growth of *E. coli, E. faecium* and *C*. *kerstersii,* and TSA medium, which is a non-selection medium, were produced.

### (2) Detection of P. aeruginosa in Human Feces Suspension Using Novel Selection Medium

As a positive control, 100 µl of the bacterial solution of *P. aeruginosa* was added to 900 µl of TSB liquid medium (final bacterial count: 10³ CFU/ml) and seeded to the plate media of NAC medium, NCC medium and TSA medium (triplicate). Moreover, as a negative control, a solution obtained by adding 100 µl of 1% Tween20-added TSB liquid medium to 900 µl of a 10× suspension of feces was seeded to the plate media of NAC medium and NCC medium. The 10× suspension of feces was obtained from a healthy individual and thus did not contain *P. aeruginosa.* Next, 100 µl of a bacterial solution of *P. aeruginosa* (after homogenization) adjusted at 10⁴ CFU/ml using 1% Tween20-added TSB liquid medium was added to 900 µl of the 10× suspension of feces (final bacterial count: 10³ CFU/ml) and thoroughly mixed. To the plate media of NAC medium and NCC medium, 100 µl taken from the mixture was seeded (triplicate) . The plate media were aerobically cultured at 37°C for 24 hours, and the numbers of colonies appeared on the media were counted. The results are shown in Table 3. The results are shown by Log₁₀ CFU/ml (average of triplicate ± standard deviation).

**[Table 3]**

| | Bacterial solution of *P. aeruginosa* (Positive control) | Human feces suspension¹⁾ (Negative control) | Human feces suspension¹⁾ + Bacterial Solution of *P. aeruginosa* |
|---|---|---|---|
| NAC medium | 2.24±0.13 | Undetectable | Undetectable |
| NCC medium | 3.51±0.03 | ND | 3.66±0.006 |
| TSA medium | 3.49±0.04 | NT | NT |

| | | | |
|---|---|---|---|
| 1) Human feces suspension: Feces which was diluted 10 times with TBS medium ND: Less than detection limit (<2.0 [Log₁₀ CFU/ml]) NT: Measurement was not conducted. Undetectable: A large number of bacteria in the feces other than *P. aeruginosa* grew, and colonies of *P. aeruginosa* could not be identified. | | | |

When the bacterial solution of *P. aeruginosa* ATCC10145^{T} was smeared on NAC medium, the detected bacterial count decreased to about 1/10 of that on TSA medium. However, the bacterial count detected on newly developed NCC medium (a medium obtained by mixing NAC medium and TSA medium at a ratio of 1:20 and adding 0.5 µg/ml of Ceftizoxime and 64 µg/ml of Cefalotin) was equivalent to that on TSA medium, which is a non-selection medium. Under the conditions in which *P. aeruginosa* was added to the feces suspension and then smeared on NAC medium, bacteria other than the target bacterium grew on the medium, and colonies of *P. aeruginosa* could not be identified. On NCC medium, however, the growth of bacteria other than the target bacterium was strongly inhibited, and *P*. *aeruginosa* alone could be detected at an equivalent bacterial count to that of the non-selection medium.

### Industrial Applicability

The selection medium for a bacterium belonging to the genus *Pseudomonas* of the present invention can specifically and accurately quantify a bacterium belonging to the genus *Pseudomonas* such as *Pseudomonas aeruginosa* and can be thus used for appropriate selection of an antibacterial agent and early therapy in medical facilities.

## Claims

1. A selection medium for a bacterium belonging to the genus *Pseudomonas* **characterized by** containing, in a basal medium, at least one third-generation cephalosporin and at least one antibiotic selected from the group consisting of a first-generation cephalosporin and a glycopeptide-based antibiotic.

2. The selection medium for a bacterium belonging to the genus *Pseudomonas* according to claim 1, wherein the third-generation cephalosporin is ceftizoxime or cefotaxime.

3. The selection medium for a bacterium belonging to the genus *Pseudomonas* according to claim 1 or 2, wherein the first-generation cephalosporin is cefalotin.

4. The selection medium for a bacterium belonging to the genus *Pseudomonas* according to any one of claims 1 to 3, wherein the glycopeptide-based antibiotic is vancomycin.

5. The selection medium for a bacterium belonging to the genus *Pseudomonas* according to any one of claims 1 to 4, wherein the basal medium is a medium mixture of NAC medium and a medium other than NAC medium which does not inhibit the growth of the bacterium belonging to the genus *Pseudomonas.*

6. The selection medium for a bacterium belonging to the genus *Pseudomonas* according to any one of claims 1 to 5, wherein the bacterium belonging to the genus *Pseudomonas* is *Pseudomonas aeruginosa.*

7. The selection medium for a bacterium belonging to the genus *Pseudomonas* according to any one of claims 1 to 6, wherein the third-generation cephalosporin is ceftizoxime, the first-generation cephalosporin is cefalotin, the glycopeptide-based antibiotic is vancomycin, the basal medium is a medium obtained by mixing NAC medium and TSA medium at a mass ratio of 1:1 to 20, the concentration of ceftizoxime in the basal medium is 0.1 µg/ml or more and less than 64 µg/ml, the concentration of cefalotin is higher than 16 µg/ml, and the concentration of vancomycin is higher than 128 µg/ml.

8. A method for detecting a bacterium belonging to the genus *Pseudomonas* **characterized by** seeding and culturing a sample in the selection medium for a bacterium belonging to the genus *Pseudomonas* according to any one of claims 1 to 7 and detecting the bacterium belonging to the genus *Pseudomonas.*

9. The method for detecting a bacterium belonging to the genus *Pseudomonas* according to claim 8, wherein the bacterium belonging to the genus *Pseudomonas* is *Pseudomonas aeruginosa.*

10. The method for detecting a bacterium belonging to the genus *Pseudomonas* according to claim 8 or 9, wherein the sample is feces.

11. A method for selecting a bacterium belonging to the genus *Pseudomonas* **characterized by** seeding and culturing a sample in the selection medium for a bacterium belonging to the genus *Pseudomonas* according to any one of claims 1 to 7 and selectively culturing the bacterium belonging to the genus *Pseudomonas.*

12. The method for selecting a bacterium belonging to the genus *Pseudomonas* according to claim 11, wherein the bacterium belonging to the genus *Pseudomonas* is *Pseudomonas aeruginosa.*

13. The method for selecting a bacterium belonging to the genus *Pseudomonas* according to claim 11 or 12, wherein the sample is feces.
